# EUROPÄISCHE PATENTSCHRIFT

(11) **EP 1 449 556 B1**
(45) Veröffentlichungstag und Bekanntmachung des Hinweises auf die Patenterteilung: **19.03.2008**
(21) Anmeldenummer: 04400005.7
(22) Anmeldetag: 12.02.2004
(51) Int. Cl.: A61M 5/32, B65D 43/02

(54) **Sammel- und Entsorgungsbehälter, insbesondere für medizinischen Abfall**
Collect and disposal container, especially for medical waste
Conteneur de collection et de déchets, spécialement pour des déchets médicaux

(30) Priorität: 22.02.2003 DE 10307773
(43) Veröffentlichungstag der Anmeldung: 25.08.2004
(73) Patentinhaber: Rigling, Heinz, D-75382 Althengstett (DE)
(72) Erfinder: Joachim, Rigling, 75382 Althengstett (DE)
(74) Vertreter: Behrmann, Niels

(56) Entgegenhaltungen:
- DE-C- 19 847 619
- US-A- 5 634 566

## Beschreibung

Die Erfindung betrifft einen Sammel- und Entsorgungsbehälter, insbesondere für medizinischen Abfall, nach dem Oberbegriff des Patentanspruchs 1.

Solche Abfallbehälter, z. B. für Kanülen von Einwegspritzen, Tupfer, Skalpelle und sonstigem medizinischen Kleinabfall, haben ein Behälterunterteil und z. B. ein vor der Ingebrauchnahme des Behälters auf unlösbare Weise darauf anzuordnendes Behälterdeckelteil mit einer durch einen Einwurföffnungsdeckel verschließbaren Abfall-Einwurföffnung.

Von deren z. B. kreisrunden Rand kann, wie z. B. aus der DE 295 14 796 U1 bekannt ist, ein Verschlusskragen hochstehen. In ihn wird zum vorübergehenden Verschluss der Einwurföffnung, z. B. für Behälterbenutzungspausen, der napfartig vertieft ausgebildete, durch ein Halteband mit dem Kragen verbundene Einwurföffnungsdeckel in eine erste, weniger tiefe Position in den Kragen eingedrückt, aus der er leicht wieder herausgezogen werden kann. Aus einer zweiten tieferen, optisch von der ersten kaum unterscheidbaren endgültigen Einwurföffnungs-Verschlussposition, kann er, meist infolge einer Verrastung, nicht mehr, zumindest nicht ohne Werkzeug, entfernt werden.

Der aus der US 5 634 566 bekannte größere Kunststoff-Abfallbehälter für Krankenhausabfall hat einen plattenförmig-flachen Behälterdeckel mit einer kreisrunden Einwurföffnung in einer muldenförmigen Vertiefung. Vom Öffnungsrand steht ein Kragen senkrecht nach unten ab. An seiner Innenseite sind zwei horizontale Reihen bildende Haltenocken nasenartig schräg nach unten vorspringend ausgebildet.

Der, wie bei Milchkannen, hohlzylindrische, unten offene und oben eine von einem vom Deckelrand hoch stehenden Kragen bündig eingerahmte Griffleiste aufweisende Verschlussdeckel hat auf seiner Innenwandung mehrere, bei Druck nach innen ausweichend ausgebildete, mit den Haltenocken zusammenwirkende Rastzungen mit nach außen abstehenden Einrastnasen.

Zum zeitweiligen Einwurföffnungs-Verschluss wird der lose Verschlussdeckel so weit in die Einwurföffnung gedrückt, bis seine Einrastnasen unterhalb der oberen Haltenocken eingerastet sind. Um ihn wieder abzunehmen wird er verdreht, bis die Einrastnasen mit entsprechenden Lücken in dieser Haltenocken-Reihe fluchten.

Zum endgültigen Einwurföffnungs-Verschluss wird der Verschlussdeckel so tief eingedrückt, bis die Einrastnasen unterhalb der unteren Haltenocken eingerastet sind, zwischen denen keine Lücken ausgebildet sind.

Ungünstig an diesem Einwurföffnungs-Verschluss ist, dass der Verschlussdeckel wegen der notwendigen horizontalen Drehbewegungen ein loses, nicht unverlierbar mit dem Behälter verbundenes Teil bildet. Seine Handhabung erfordert nicht nur eine gewisse Vertrautheit mit dem für den zeitweiligen und endgültigen Verschluss vorgesehenen Deckel-Arretierungskonzept, sondern, bei der vorgesehenen Behältergröße und der dadurch bedingten jeweiligen Materialdicken, auch einen nicht unerheblichen Kraftaufwand mit einer dafür ungünstig zu betätigenden Griffleiste.

Zum zeitweiligen wie auch endgültigen Verschluss der Einwurföffnung kann auf der Oberseite des Behälterdeckelteils auch ein den Rand der Einwurföffnung überdeckender, auf- und zuklappbar angeordneter Einwurföffnungsdeckel vorgesehen sein, wie z. B. aus der DE 198 47 619 C1 bekannt ist. Er kann ebenfalls für diese beiden Verschlusszustände in zwei, optisch auch kaum unterscheidbare, verschieden tiefe Positionen auf einem gleicherweise vom Rand der Einwurföffnung hochstehenden Verschlusskragen, diesen umgreifend, aufgedrückt und, ebenso von außen unzugänglich, verrastet werden.

Es hat sich nun gezeigt, dass es nicht nur ein Grundbedürfnis gibt nach einem - während des immer wieder unterbrochenen Abfallsammelns - bequem handzuhabenden zeitweiligen Verschluss der Abfall-Einwurföffnung solcher Behälter mit Hilfe eines Einwurföffnungsdeckels und einem einfach herbeizuführenden sicheren endgültigen Verschluss der Einwurföffnung. Es besteht darüber hinaus auch ein Bedürfnis, allein durch flüchtigen Augenschein sofort erkennen zu können, ob die Einwurföffnung eines solchen Behälters endgültig verschlossen ist und dieser somit zur Entsorgung gegeben werden kann.

Das diesen Bedürfnissen entsprechende, der Erfindung zugrunde liegende Problem wird mit den Merkmalen des Patentanspruchs 1 gelöst. Vorteilhafte Weiterbildungen sind Gegenstand der Unteransprüche.

Dadurch, dass der Einwurföffnungsdeckel aus zwei Teilen gebildet ist, nämlich einem für den endgültigen Verschluss der Einwurföffnung bestimmen, die plattenartige Ausgestaltung aufweisenden Deckelelement mit an die Kontur des Randes und der lichten Weite der Einwurföffnung angepasster Ausgestaltung mit einem randseitig nach unten abstehenden Verschlusskragen, an dessen Außenseite, seinen Umfang vergrößernd, seitwärts federnd abstehend und umfangsverringernd an diese Außenseite herandrückbar die Einrastelemente ausgebildet sind, und einem das Deckelelement bis zum endgültigen Verschließen der Einwurföffnung haltenden, mit ihm auf- und zuklappbar auf dem Behälterdeckelteil angeordneten Halteelement mit einer sein Aufliegen auf dem Behälterdeckelteil im Randbereich der Einwurföffnung ermöglichenden, das Deckelelement randseitig rahmenförmig umschließenden Ausgestaltung, dessen Einrastelemente hierbei unter Spannung derart an der Innenseite des Halteelements anliegen, dass es bei auf dem Behälterdeckelteil aufliegenden Halteelement aus diesem durch einen Kraftaufwand nach unten herausdrückbar ist, und dass der Rand der Einwurföffnung als Verschlusselement einen zumindest abschnittsweise nach unten abstehend umlaufenden und in die Einwurföffnung vorstehenden annähernd U-förmigen Sitz für den Verschlusskragen des nach unten herausgedrückten Deckelelements aufweist mit einem für den endgültigen Verschluss der Einwurföffnung mit den Einrastelementen des Deckelelements zusammenwirkenden Sitz-Verriegelungselement, wird Folgendes erreicht:

Zum Einwerfen von Abfall in den Behälter wird der Einwurföffnungsdeckel aufgeklappt, wodurch die Einwurföffnung freiliegt. Zu ihrem vorübergehenden Verschluss, z. B. für Benutzungspausen des Behälters, wird er lediglich wieder zugeklappt.

Zum endgültigen, d. h. nicht wieder rückgängig zu machenden Verschluss der Einwurföffnung, kann erfindungsgemäß im zugeklappten Zustand des Einwurföffnungsdeckels sein dafür vorgesehenes Deckelelement, z. B. mit den Daumen beider Hände, nach unten aus seinem Haltelement herausgedrückt werden. Hierfür ist lediglich der Widerstand der es darin haltenden Einrastelemente des Verschlusskragens zu überwinden, wobei das Haltelement vom Randbereich der Einwurföffnung, auf dem es aufliegt, abgestützt wird.

Bei diesem Herausdrücken tritt gleichzeitig der randseitige Verschlusskragen des Deckelelements, aufgrund der Anpassung letzteren an die Kontur des Randes und die lichte Weite der Einwurföffnung, nahe dieses Randes sukzessiv in den daran ausgebildeten Verschlusskragen-Sitz ein, bis er seine vorgesehene, dem endgültigen Verschluss der Einwurföffnung durch das Deckelelement entsprechende Position an oder, je nach Ausgestaltung, ggf. auch in diesem Sitz eingenommen hat.

Gleichzeitig damit werden auch die Einrastelemente des Verriegelungskragens zusammen mit dem Verriegelungselement des Sitzes, z. B. durch eine nach außen verdeckte Verrastung, wirksam, wodurch das Deckelelement unlösbar in dieser, die Einwurföffnung endgültig verschließenden Position gehalten ist.

Durch die von außen unzugängliche Ausgestaltung dieser Einrastelemente und des Sitz-Verriegelungselements ist der endgültige Verschluss der Einwurföffnung sehr sicher, zumal dann sich z. B. auch die obere Kante des Randes des Deckelelements auf der Höhe des Randes der Einwurföffnung bzw. der es umgebenden Oberseite des Behälterdeckelteils befinden kann und so keine Angriffsmöglichkeiten für sein Lösen aus dieser Verschlussanordnung bietet.

Nachdem das nach unten bis in seine Endposition gedrückte Deckelelement durch seinen Verschlusskragen bzw. dessen Einrastelementen vom Sitz gehalten ist, liegt statt eines flächigen Einwurföffnungsdeckels nun nur noch das vorher eine Art Rahmen für das Deckelelement bildende Halteelement im Randbereich der Einwurföffnung auf der Oberseite des Behälterdeckelteils auf. Durch dieses somit "leere" Halteelement fällt der Blick unbehindert direkt auf das sich vertieft darunter befindende, die Einwurföffnung verschließende Deckelelement. Dadurch ist mit einem Blick sofort erkennbar, dass die Einwurföffnung des betreffenden Behälters endgültig verschlossen ist und dieser somit der Entsorgung zugeführt werden kann.

Da das jetzt "leer" auf der Oberseite des Behälterdeckelteils aufliegende Halteelement weiterhin klappbar ist, kann es hochgeklappt und, z. B. als Griff, zum Tragen des Behälters benutzt werden. Dabei signalisierte es in dieser hochgeklappten Stellung besonders auffällig den endgültigen Verschluss der Einwurföffnung.

Mit Vorteil ist die Einwurföffnung in einer flachen, muldenartig in der Oberseite des Behälterdeckelteils ausgebildeten Vertiefung angeordnet, wobei deren Tiefe und die Höhe des Halteelements des Einwurföffnungsdeckels aneinander angepasst sind. Dadurch kann der Einwurföffnungsdeckel beim Zuklappen vollständig von der Vertiefung aufgenommen werden, steht also randseitig nicht über die Oberseite des Behälterdeckelteils über. Auf diese Weise ist er nicht nur vor evtl. Einwirkungen aus dem Umfeld besser geschützt, sondern ist auch schon der nur zeitweilige Verschluss der Einwurföffnung durch den sie hierzu überdeckenden, randseitig in ihrem Randbereich aufliegenden und von der Seitenwandung der Vertiefung geschützt umgebenen Einwurföffnungsdeckels sicherer.

Darüber hinaus erhöht eine solche Vertiefung vor allem aber die Sicherheit des endgültigen, durch das Eindrücken des Deckelelements in die Einwurföffnung, bzw. seines Verschlusskragens in den Sitz, herbeigeführten Einwurföffnungsverschlusses. Denn eine solche Vertiefung erschwert zusätzlich einen von der Seite versuchten Zugang zu dem dann gegenüber der Oberseite des Behälterdeckelteils tiefer liegenden, im Sitz verriegelt gehaltenen Deckelelement einschließlich der es darin haltenden Einrastelementen und somit auch einen Versuch, letztere zu überwinden.

Vorzugsweise weisen die Vertiefung, zumindest überwiegend, und - daran angepasst - auch die Einwurföffnung und der Einwurföffnungsdeckel eine elliptische Form auf. Diese Form führt einerseits zu einer großen Stabilität des dann ebenfalls elliptischen Deckelelements des Einwurföffnungsdeckels und andererseits ermöglicht sie für ein unbehindertes Einwerfen von Abfall in den Behälter eine optimale lichte Weite der Einwurföffnung. Insbesondere dann, wenn sich die Ellipsen-Hauptachse bei einem eckigen, z. B. quadratischen, Behälterdeckelteil bis hin zu zwei gegenüberliegenden Seitenrändern erstreckt.

Dieser Vorteil wird auch durch eine z. B. in der Einwurföffnung segmentartig ausgebildete Materialbrücke mit Abzugs- und/oder Abdreh-Ausgestaltungen für Kanülen bzw. Kanülenhalter nicht wesentlich beeinträchtigt, im Gegensatz etwa zu einer kreisförmigen Einwurföffnung entsprechender Querschnittsfläche.

Im Hinblick auf eine sozusagen glatte Oberfläche des Behälterdeckelteils, um z. B. bei geschlossener Einwurföffnung diese Behälter übereinander stapeln zu können, sind die den Einwurföffnungsdeckel haltenden Gelenkausgestaltungen vorzugsweise in einer die - für den Einwurföffnungsdeckel vorgesehene - Vertiefung seitwärts vergrößernden eckigen, z. B. einen Teil eines Rechtecks bildenden Erweiterung vorgesehen, ohne über sie überzustehen.

Mit Vorteil sind als Einrastelemente des Verschlusskragens eine Anzahl voneinander beabstandeter Einrastzungen außen am Verschlusskragen des Deckelelements, vom unteren Endbereich schräg nach oben gerichtet abstehend, vorgesehen. Mit diesen elastischen Zungen kann nicht nur das Deckelelement bis zum endgültigen Verschluss der Einwurföffnung sicher im entsprechend daran angepasst ausbildbaren Halteelement gehalten werden, das hierfür einen z. B. zwischen seiner Ober- und Unterkante entsprechend an die schräge Ausrichtung dieser Einrastzungen angepasst ausgebildeten Wandungsabschnitt aufweist, an den sich die Einrastzungen des im Halteelement angeordneten Deckelelements mit Federkraft anlegen können.

Solche Zungen ermöglichen auch ein leichtes nach unten gerichtetes Herausdrücken des Deckelelements aus dem Halteelement, wofür sie sich - umfangsverringernd - soweit an den Verschlusskragen herandrücken lassen, bis ein Herausgleiten des Deckelelements aus dem Halteelement möglich ist.

Anhand von schematischen Zeichnungen wird nachfolgend ein für medizinischen Abfall bestimmter Sammel- und Entsorgungsbehälter, kurz Behälter, in einer bevorzugten Ausführung der Erfindung beschrieben, wobei sich weitere Einzelheiten und Vorteile der Erfindung ergeben.

Es zeigt:
- Fig. 1: eine perspektivische Teilansicht des Behälters mit aufgeklapptem Einwurföffnungsdeckel,
- Fig. 2: eine weitere perspektivische, teilweise geschnittene Teilansicht des Behälters nach Fig. 1,
- Fig. 3: in einer verkleinerten perspektivischen Teilansicht den Behälter nach Fig. 1 mit zugeklapptem Einwurföffnungsdeckel zum zeitweiligen Verschluss der Einwurföffnung,
- Fig. 4: in einer vergrößerten perspektivischen Ansicht einen Längsschnitt durch den Behälter nach Fig. 3,
- Fig. 5: in einer verkleinerten perspektivischen Teilansicht den Behälter nach den vorhergehenden Figuren mit endgültig verschlossener Einwurföffnung,
- Fig. 6: in einer vergrößerten perspektivischen Ansicht einen Längsschnitt durch den Behälter nach Fig. 5 und
- Fig. 7: in einer vergrößerten perspektivischen Teilansicht den Behälter nach Fig. 5 mit hochgeklapptem "leeren" Halteelement.

Der in den Figuren jeweils nur zum Teil gezeigte Behälter mit einem Fassungsvermögen von z. B. 4 oder 7 Litern hat ein sich konisch nach unten verjüngendes Behälterunterteil 1 und ein sich konisch nach oben verjüngendes Behälterdeckelteil 2, jeweils quadratischen Querschnitts. Sie sind durch eine Rastausgestaltung im Verbindungsrandbereich unlösbar und flüssigkeitsdicht miteinander verbunden. Zum Entsorgen des Behälters samt Inhalts durch Verbrennen bestehen sie aus einem dafür geeigneten Kunststoff.

Um den Behälter, z. B. bis zur seiner Entsorgung, d. h. während der Dauer des Befüllens, bequem tragen zu können, ist an seinem Unterteil ein Tragegriff 3 ausgebildet.

Auf der Oberseite des eine Kantenlänge von z. B. 13 cm aufweisenden Behälterdeckelteils 2 ist eine flache muldenartige Vertiefung 10 in einer für eine optimale Größe in Bezug auf die quadratische Deckfläche des Behälterdeckelteils 2 überwiegend elliptischen Form ausgebildet. Die Vertiefung 10 nimmt die gesamte Breite des Behälterdeckelteils 2 ein und reicht im Bereich des vorderen Ellipsen-Nebenscheitels bis an dessen Vorderkante heran. Sie ist durch eine eckige, einen Teil eines Rechtecks bildende Erweiterung 11 über die Mitte des Behälterdeckelteils 2 hinaus nach hinten vergrößert. Ihre Seitenwandung 12 verläuft querschnittsverengend schräg einwärts gerichtet nach unten.

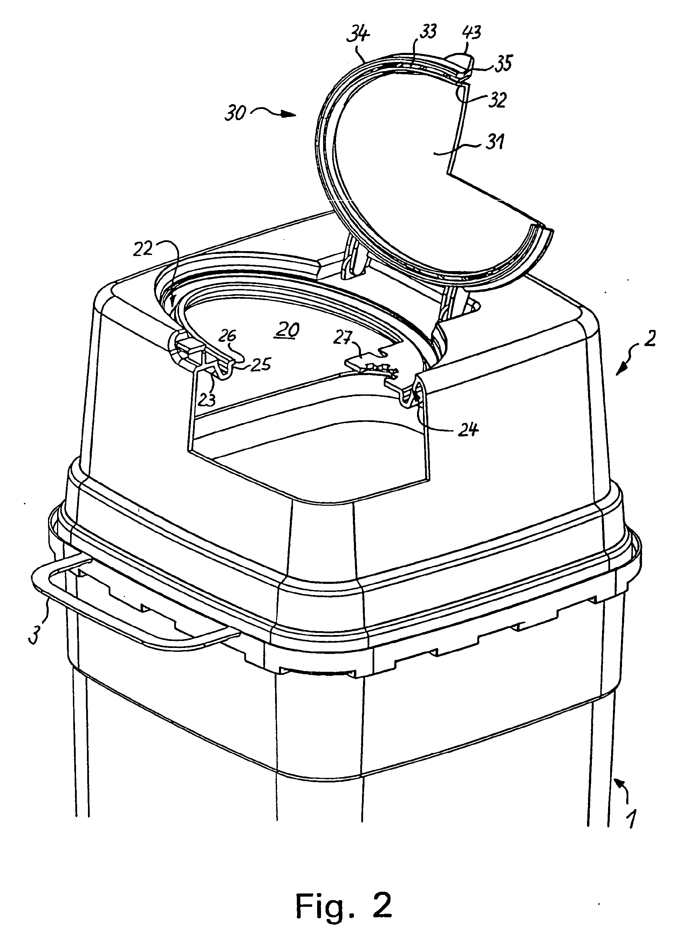

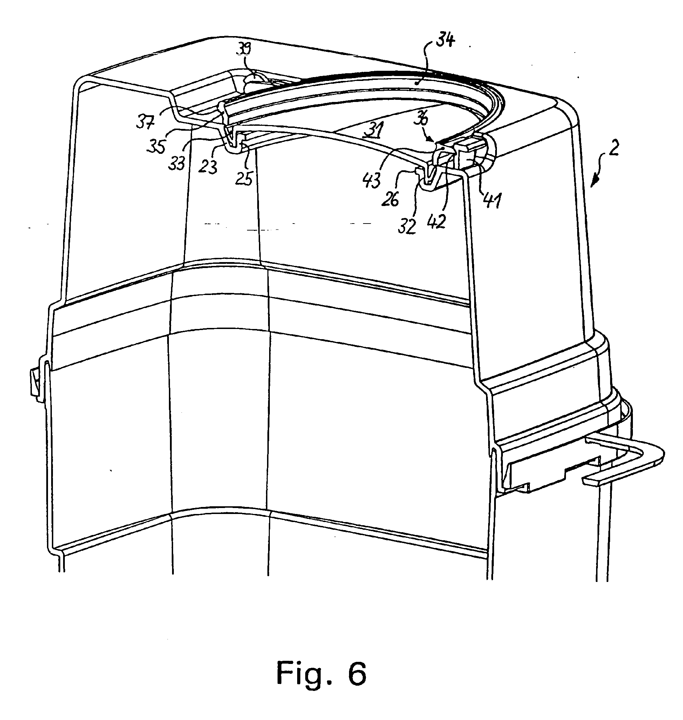

Am Grund der elliptischen Vertiefung 10 ist als Abfall-Einwurföffnung 20 eine für eine möglichst große lichte Weite, daran angepasst, ebenfalls elliptische Ausnehmung vorgesehen. Von ihrem Rand 21 steht ein durch Materialverformung annähernd U-förmig ausgebildeter Sitz 22, nach unten weisend umlaufend, in die Einwurföffnung 20 vor (Fig. 2, 6).

Wie in den Schnittansichten nach Fig. 2, 4, 6 im Detail erkennbar, was auch für die nachfolgenden Ausführungen gilt, wird im elliptischen Bereich der Vertiefung 10 der äußere, in Anpassung an die schräg nach oben gerichtet außen vom Verschlusskragen 32 abstehenden Einrastzungen 33 entsprechend schräg verlaufende Schenkel 23 des Sitzes 22 von einer Verlängerung der ebenfalls schräg einwärts verlaufenden Seitenwandung 12 der Vertiefung 10 nach unten gebildet. Im Bereich von deren eckigen Erweiterung 11 ist er dagegen durch eine entsprechende Verformung deren Bodens 13 gebildet.

Auf der Innenseite des äußeren Schenkels 23 steht vom oberen Rand, der sich auf dem Niveau des von der Einwurföffnung 20 eingenommenen Grundes der Vertiefung 10 bzw. der Oberseite des Bodens 13 der eckigen Erweiterung 11 befindet, eine nach innen gerichtete schmale Verriegelungslippe 24 ab. Am oberen Ende des kürzeren inneren Schenkels 25 ist eine nach innen in die Einwurföffnung vorspringende Verstärkungsrippe 26 ausgebildet.

Ein rechter Teilbereich der Einwurföffnung 20 ist durch eine segmentförmig ausgebildete ebene Materialbrücke 27 mit Ausgestaltungen 28 bzw. 29 zum Abziehen von Kanülen bzw. zum Abschrauben von Kanülenhaltern von Spritzenkörpern überwiegend verschlossen.

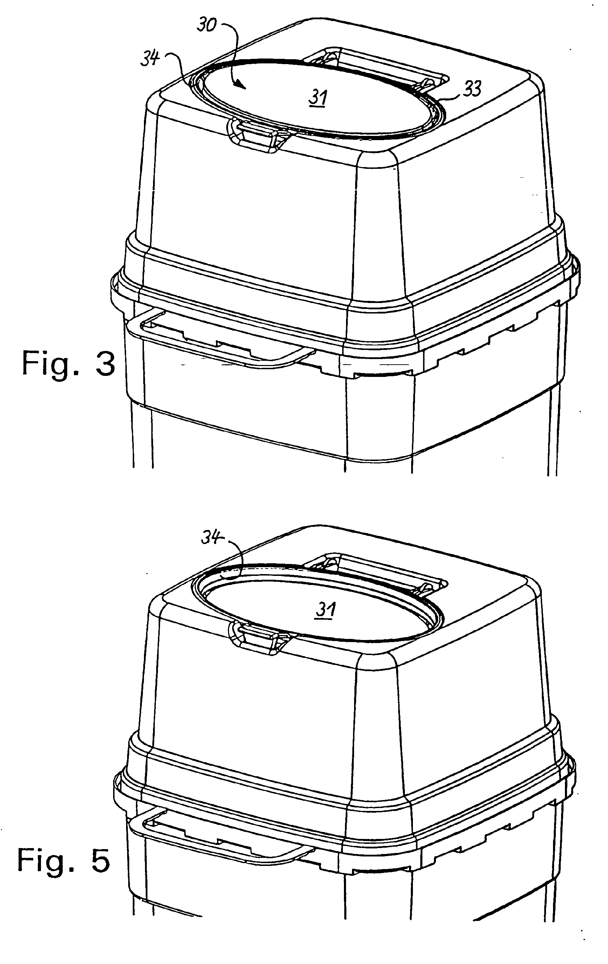

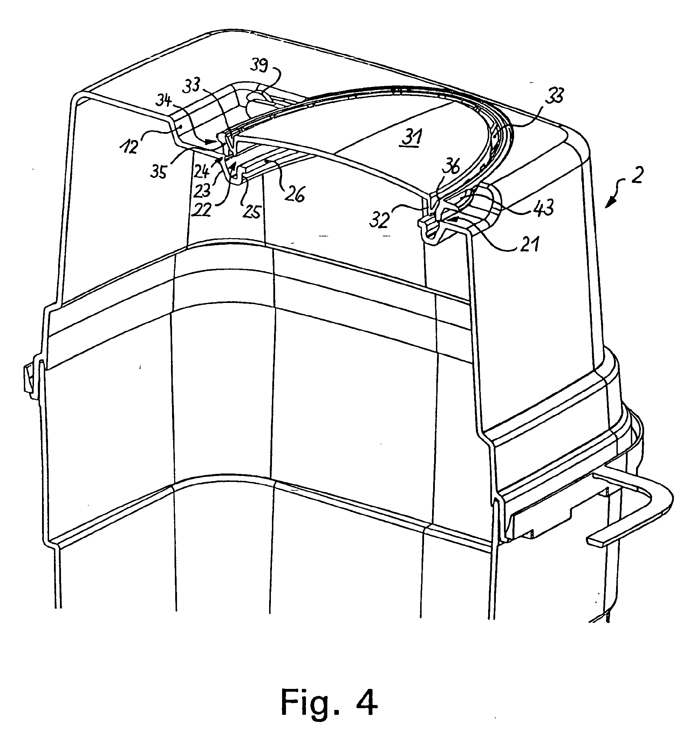

Zum zeitweiligen Verschluss der Einwurföffnung 20 (Fig. 3, 4) ist in Anpassung an die elliptische Form der Vertiefung 10 bzw. des Randes 21 der Einwurföffnung 20 ein ebenfalls elliptischer, aus zwei Teilen gebildeter Einwurföffnungsdeckel 30 vorgesehen. Bei dem einen Teil handelt es sich um ein plattenartiges, aus Stabilitätsgründen zur Mitte hin leicht nach oben gewölbtes Deckelelement 31 zum Verschluss der Einwurföffnung 20 mit dieser gegenüber geringfügig kleineren Abmessungen. Es weist einen von seinem Rand senkrecht nach unten abstehenden Verschlusskragen 32 auf. Dessen innerer Umfang und der äußere Umfang des vom inneren Schenkel 25 des Sitzes 22 gebildeten Ringes sind aneinander angepasst.

Auf der Außenseite des Verschlusskragens 32 sind als Einrastelemente für die Arretierung des Deckelelements 31 in seiner die Einwurföffnung 20 endgültig verschließenden Anordnung, das ist im Sitz 22, eine Vielzahl von jeweils geringfügig voneinander beabstandeter Einrastzungen 33 ausgebildet. Sie stehen in einem geringen Abstand vom unteren Rand des Verschlusskragens 32 bis in die Nähe des Niveaus der oberen Randkante des Deckelelements 31 schräg nach oben gerichtet elastisch vom Verschlusskragen 32 ab.

Bei dem anderen Teil des Einwurföffnungsdeckels 30 handelt es sich um ein in der Höhe der Tiefe der Vertiefung 10 entsprechendes, das Deckelelement 31 rahmenartig umschließendes Halteelement 34. Es hat einen, in Anpassung an die außen vom Verschlusskragen 32 schräg abstehend angeordneten Einrastzungen 33, zwischen seinem oberen und unteren Ende querschnittsverengend entsprechend schräg einwärts gerichtet verlaufenden Wandungsabschnitt 35.

Am oberen Rand des Halteelements 34 ist auf der Innenseite eine nach innen abstehende Rastlippe 36 und auf der Außenseite in einem Abstand unterhalb des oberen Randes eine umlaufende Verstärkungsrippe 37 vorgesehen.

Der Einwurföffnungsdeckel 30 wird durch das Eindrücken des Deckelelements 31 von oben her in das Halteelement 34 gebildet. Hierbei werden die Einrastzungen 33 beim Entlanggleiten an der Rastlippe 36 des Halteelements 34 zunächst an den Verschlusskragen 32 herangedrückt und springen danach in ihre in Fig. 4 gezeigte Raststellung. Sie liegen dann unter Spannung seitlich am schrägen Wandungsabschnitt 35 des Halteelements 34 und mit dem Rand ihrer freien Enden an der Unterseite der Rastlippe 36 an. Auf diese Weise ist das Deckelelement 31 vom Halteelement 34 gegen ein selbsttätiges Lösen aus ihm nach oben wie auch nach unten gesichert gehalten.

Vom Halteelement 34 stehen zwei Halterungen 38 seitwärts gerichtet ab. Sie sind von jeweils zwei geringfügig voneinander beabstandeten Haltezungen gebildet, die jeweils nahe ihres freien, sich verjüngenden Endes durch einen Achssteg mit rundem Querschnitt miteinander verbunden sind. Die Achsstege sind in entsprechende Ausnehmungen in zwei vom Boden 13 der Erweiterung 11 der Vertiefung 10 senkrecht bis auf das Niveau der Oberseite des Behälterdeckelteils 2 hochstehend ausgebildeten Haltenasen 39 eingerastet. Dadurch ist der Einwurföffnungsdeckel 30 um eine zur Oberseite des Behälterdeckelteils 2 parallele Achse schwenk- bzw. klappbar gehalten.

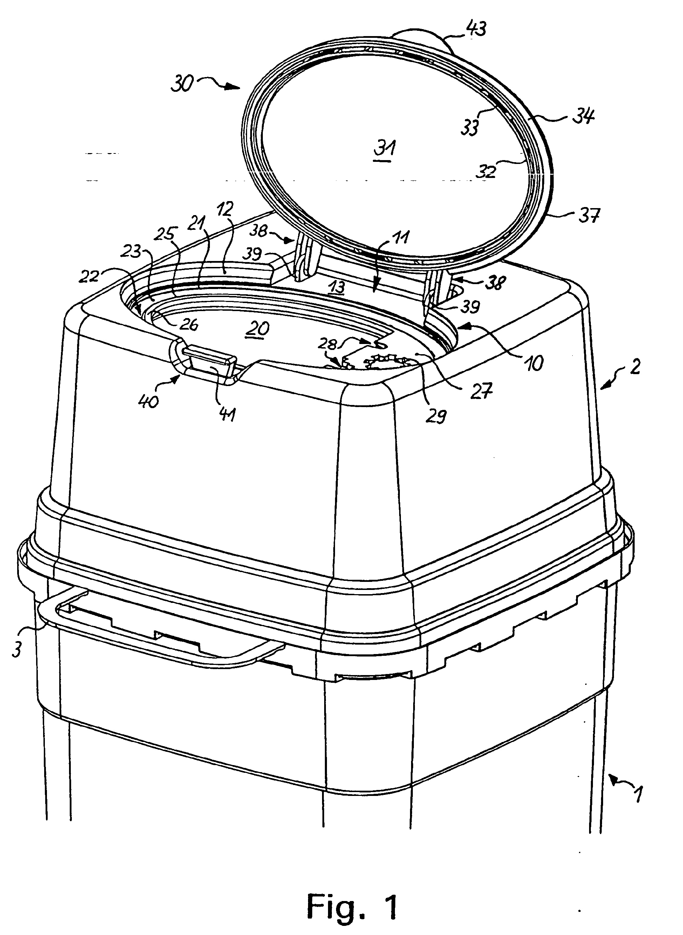

Zum zeitweiligen Verschluss der Einwurföffnung 20 durch ihr Abdecken, z. B. während einer Benutzungspause des Behälters, wird der Einwurföffnungsdeckel 30 zugeklappt, d. h. aus seiner in Fig. 1 bzw. 2 gezeigten Stellung in die in Fig. 3 bzw. 4 gezeigte Stellung heruntergeklappt. Er liegt dann (Fig. 4), überwiegend von der Seitenwandung 12 der Vertiefung 10 umgeben und so gegen evtl. schädigende Einwirkungen von außen geschützt, mit einem schmalen äußeren Abschnitt des unteren Randes des Halteelements 34 auf der oben innen vom äußeren Schenkel 23 des annähernd U-förmigen Sitzes 22 vorspringenden Verriegelungslippe 24 auf. Dabei schließt sein oberer Rand im Bereich der elliptischen Vertiefung 10 bündig mit deren oberen Randkante ab und steht so randseitig nicht über die Vertiefung 10 nach oben über.

Durch die schräg nach innen gerichtet, also querschnittsverengend verlaufende Seitenwandung 12 der Vertiefung 10 in ihrem elliptischen Bereich wird der Einwurföffnungsdeckel 30 in Verbindung mit dem ebenfalls schrägen Verlauf des Wandungsabschnitts des Halteelements beim Herunterklappen in seine zeitweilige Verschlussstellung (Fig. 3, 4) in der Vertiefung 10 sozusagen zentriert.

Zur Arretierung des Einwurföffnungsdeckels 30 in dieser die Einwurföffnung 20 zeitweilig verschließenden Stellung ist im Bereich der oberen Randkante der als Vorderseite des Behälterdeckelteils 2 vorgesehenen Behälterseite eine Arretierungsausnehmung 40 mit einer nach außen auslenkbaren Arretierungsraste 41 (nicht gezeigt in Fig. 4) ausgebildet. Letztere weist eine Arretierungskerbe 42 auf ihrer Innenseite auf (Fig. 2 und 6). In diese ist im heruntergeklappten Zustand des Einwurföffnungsdeckels 30 (Fig. 3) ein an dessen Halteelement 34 entsprechend nach außen abstehend ausgebildete Arretierungslasche 43 eingerastet. Stattdessen kann sie auch in eine entsprechende Ausnehmung in der Arretierungsraste 41 eingreifen.

Da der Einwurföffnungsdeckel 30 in seiner zeitweiligen Verschlussstellung (Fig. 3 bzw. 4) überwiegend von der Seitenwandung 12 der Vertiefung 10 umrandet ist und nicht nach oben übersteht und damit auch zum Auf- bzw. Hochklappen nicht unmittelbar zugänglich ist, ist auch seine von der Arretierungsraste 41 gehaltene Arretierungslasche 43 nach dem Lösen ggf. weiterhin schlecht zugänglich. Damit er dennoch auf einfache Weise auf- bzw. hochklappbar ist, kann z. B. eine hier nicht gezeigte, mit einer der beiden Halterungen 38 des Einwurföffnungsdeckels 30 zusammenwirkende Feder, z. B. eine Schenkelfeder, vorgesehen werden. Sie wird beim Zuklappen des Einwurföffnungsdeckels 30 gespannt. Wenn dann die Arretierungsraste 41 nach vorn gezogen wird, springt der Einwurföffnungsdeckel 30 von selbst in die in Fig. 1 und 2 gezeigte Stellung, in der er dann auch von der Feder gehalten wird. Somit kann Abfall in den Behälter eingeworfen werden, ohne dass der Einwurföffnungsdeckel 30 etwa von selbst zuklappt.

Eine solche federkraftbetätigte Aufklapphilfe für den Einwurföffnungsdeckel 30 kann die Benutzung dieses Behälters nicht nur bequemer, sondern auch sicherer machen. Denn sie kann dazu führen, dass der Einwurföffnungsdeckel 30 aufgrund dieses handhabungsvereinfachend selbsttätigen Aufklappens anschließend wieder vom Behälterbenutzer heruntergeklappt wird, da er hierbei mittels seiner von selbst einrastenden Arretierungsraste 41 auch gleichzeitig wieder arretiert wird. So würde die Einwurföffnung 20 immer wieder vorübergehend, ggf. sogar nach jedem Einwerfen von Abfall in den Behälter, verschlossen und damit die Sicherheit der Benutzung dieser Behälter erhöht.

Zum endgültigen Verschluss der Einwurföffnung 20 (Fig. 5, 6, 7) wird bei auf die Einwurföffnung 20 heruntergeklappten, durch die Arretierungslasche 43 und die Arretierungsraste 41 in dieser Position arretierten Einwurföffnungsdeckel 30 (Fig. 3, 4) ein, z. B. mit den Daumen beider Hände, nach unten gerichteter Druck auf das Deckelelement 31 ausgeübt. Dabei wird das von der schräg verlaufenden Seitenwandung 12 der Vertiefung 10 zentrierte Halteelement 34 am unteren Rand von der Verriegelungslippe 24 abgestützt. Durch den ausgeübten Druck werden die Einrastzungen 33 an den Verschlusskragen 32 herangedrückt, so dass sich das Deckelelement 31 infolge der sich dadurch bei ihm gewissermaßen einstellenden Umfangsverringerung nach unten aus dem Halteelement 34 herausdrücken lässt.

Bei aufrechterhaltenem Druck tritt dann sukzessive der Verschlusskragen 32 des Deckelelements 31, quasi von den Einrastzungen 33 geführt, in den gewissermaßen wie eine annähernd U-förmige Nut umlaufenden Sitz 22 ein. Beim Erreichen seiner vorgesehenen Endposition darin rasten die Einrastzungen 33 unter die Verriegelungslippe 24 am Rand des äußeren Schenkels 23 des Sitzes 22 ein und halten so das Deckelelement 31, die Einwurföffnung 30 endgültig verschließend, irreversibel im Sitz 22 am Grund der Vertiefung 10 fest (Fig. 5, 6).

Hierfür ist der innere Schenkel 25 des Sitzes 22 in Anpassung an die Materialdicke des Deckelelements 31 entsprechend kürzer ausgebildet als der äußere Schenkel 23. Dadurch kann wiederum das Deckelelement 31, sofern das im Hinblick auf die beabsichtigte Signalwirkung der vertieften endgültigen Verschlussanordnung des Deckelelements 31 überhaupt von Bedeutung sein sollte, eine relativ geringe Höhe, bzw. die Vertiefung 10 eine dementsprechend geringe Tiefe aufweisen. Seine obere Randkante kann sich so, gut geschützt gegen Manipulationen, auf der Höhe des Randes 21 der Einwurföffnung befinden.

Am Grund des Sitzes 22 kann eine mit dem unteren Rand des Verschlusskragens 32 zusammenwirkende Dichtung vorgesehen werden, um die Flüssigkeitsdichtigkeit des endgültigen Verschlusses der Einwurföffnung 20 zu ermöglichen. Dies kann aber auch bereits durch den aufgrund der Federspannung der Einrastzungen 33 mit seiner Innenseite an die Innenseite des inneren Schenkels 25 gedrückten Verschlusskragen 32 gegeben sein.

Durch diese nun bei endgültigem Verschluss der Einwurföffnung 20 gegenüber der Oberseite des Behälterdeckelteils 2 auffällige tiefere Lage des Deckelelements 31 am Grund der Vertiefung 10 ist sofort auf einen Blick erkennbar, dass der betreffende Behälter endgültig für die Entsorgung verschlossen ist.

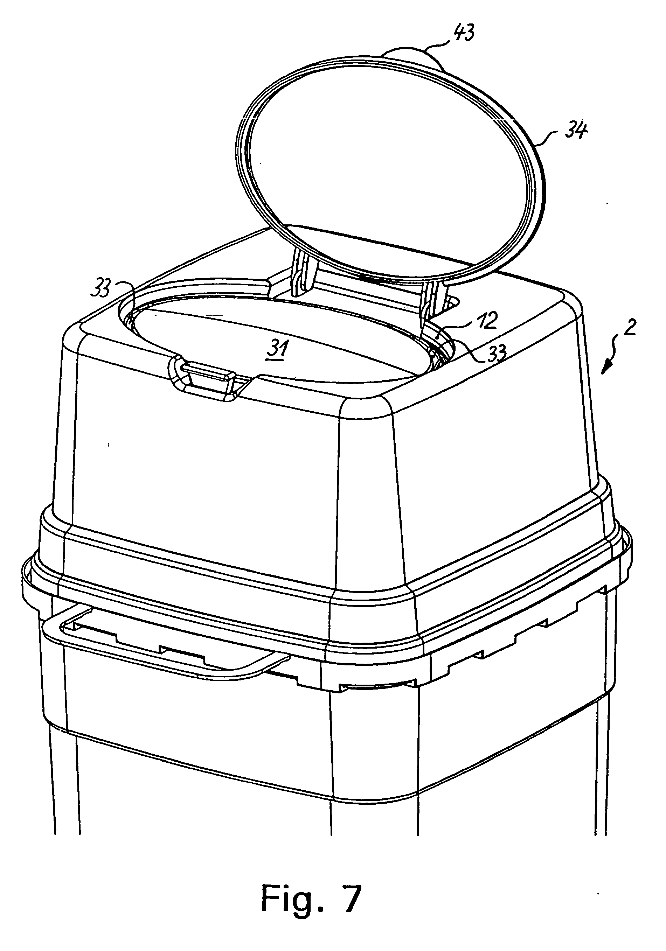

Dies kann noch zusätzlich dadurch verdeutlicht bzw. richtiggehend signalisiert werden, dass das dann "leere" Halteelement 34 in die in Fig. 7 gezeigte Stellung hochgeklappt wird bzw. man es federkraftbetätigt durch Ausrasten der es in der Vertiefung 10 haltenden Arretierungsraste 41 (Fig. 5) hochspringen lässt. In dieser Stellung kann es dann sogar, bei entsprechend stabiler Ausführung von Halteelement 34, der Halterungen 38 bzw. Haltenasen 39, als Griff zum bequemen Tragen auch eines voll gefüllten Behälters dienen.

Mit der Idee, den Einwurföffnungsdeckel 30 der Einwurföffnung 20 eines Abfall-Sammel- und Entsorgungsbehälters zweiteilig mit einem Halteelement 34 und einem von diesem für den endgültigen Verschluss der Einwurföffnung 20 nach unten herauslösbar gehaltenen Deckelelement 31 auszubilden, kann ein optisch ansprechend gestaltbarer, zum zeitweiligen Verschließen der Einwurföffnung 20 durch deren ledigliches Abdecken bequem handzuhabender, für deren für die Behälterentsorgung endgültigen Verschluss gegen Manipulation dagegen extrem sicherer Verschluss geschaffen werden. Das sich dann gegenüber dem Halteelement 34, vorzugsweise auch gegenüber der Oberseite des Behälterdeckelteils 2, in einer vertieften und verriegelten Position im Behälterdeckelteil 2 befindende, die Einwurföffnung 20 irreversibel verschließende Deckelelement 31 zeigt so auf auffällige Weise an, dass der betreffende Behälter endgültig für die Entsorgung verschlossen ist, ggf. noch zusätzlich signalisiert durch das dann "leere" Halteelement 21 in einer auffällig hochstehenden Stellung.

### Bezugszeichenliste

- 1: Behälterunterteil
- 2: Behälterdeckelteil
- 3: Tragegriff

- 10: Vertiefung
- 11: Erweiterung
- 12: Seitenwandung
- 13: Boden

- 20: Einwurföffnung
- 21: Rand
- 22: Verschlusselement, Sitz
- 23: äußerer Schenkel
- 24: Sitz-Verriegelungselement, Verriegelungslippe
- 25: innerer Schenkel
- 26: Verstärkungsrippe
- 27: Materialbrücke
- 28: Kanülen-Abzugsausgestaltung
- 29: Kanülenhalter-Abdrehausgestaltung

- 30: Einwurföffnungsdeckel
- 31: Deckelelement
- 32: Verschlusskragen
- 33: Einrastelemente, Einrastzungen
- 34: Halteelement
- 35: Wandungsabschnitt
- 36: Rastlippe
- 37: Verstärkungsrippe
- 38: Halterung
- 39: Haltenase

- 40: Arretierungsausnehmung
- 41: Arretierungsraste
- 42: Arretierungsrastkerbe
- 43: Arretierungsrastlasche

## Patentansprüche

1. Sammel- und Entsorgungsbehälter, insbesondere für medizinischen Abfall, mit einem Behälterunterteil (1) und einem Behälterdeckelteil (2) mit einer Einwurföffnung (20) und einem den Rand (21) der Einwurföffnung (20) überdeckenden, auf- und zuklappbar angeordneten plattenartigen Einwurföffnungsdeckel (30), wobei im Randbereich von Einwurföffnung (20) und Einwurföffnungsdeckel (30) jeweils zusammenwirkende Verschluss- und Einrast-Elemente (22, 33) für den endgültigen, von außen unzugänglichen Verschluss der Einwurföffnung(20) vorgesehen sind,
**gekennzeichnet zumindest dadurch,**
- dass der Einwurföffnungsdeckel (30) aus zwei Teilen gebildet ist:
a) einem für den endgültigen Verschluss der Einwurföffnung (20) bestimmten, eine plattenartige Ausgestaltung aufweisenden Deckelelement (31) mit einer an die Kontur des Randes (21) und der lichten Weite der Einwurföffnung (20) angepassten Ausgestaltung mit einem randseitig nach unten abstehenden Verschlusskragen (32), an dessen Außenseite, seinen Umfang vergrößernd, seitwärts federnd abstehend und umfangsverringernd an diese Außenseite herandrückbar die Einrastelemente (33) ausgebildet sind, und
b) einem das Deckelelement (31) bis zum endgültigen Verschließen der Einwurföffnung (20) haltenden, mit ihm auf- und zuklappbar auf dem Behälterdeckelteil (2) angeordneten Halteelement (34), welches eine sein Aufliegen auf dem Behälterdeckelteil (2) im Randbereich der Einwurföffnung (20) ermöglichende, das Deckelelement (31) randseitig rahmenförmig umschließende Ausgestaltung hat, wobei die Einrastelemente (33) des Deckelelementes hierbei unter Spannung derart an der Innenseite des Halteelements (34) anliegen, dass das Deckelelement (31) bei auf dem Behälterdeckelteil (2) aufliegendem Halteelement (34) aus diesem durch einen Kraftaufwand nach unten herausdrückbar ist, und
- dass der Rand der Einwurföffnung (20) als Verschlusselement einen zumindest abschnittsweise nach unten abstehend umlaufenden und in die Einwurföffnung (20) vorstehenden annähernd U-förmigen Sitz (22) für den Verschlusskragen (32) des nach unten herausgedrückten Deckelelements (31) aufweist, wobei der Sitz ein für den endgültigen Verschluss der Einwurföffnung (20) mit den Einrastelementen (33) des Deckelelements (31) zusammenwirkendes Sitz-Verriegelungselement (24) aufweist.

2. Behälter nach Anspruch 1, **dadurch gekennzeichnet, dass** die Einwurföffnung (20) in einer flachen, muldenartig in der Oberseite des Behälterdeckelteils (2) ausgebildeten Vertiefung (10) angeordnet ist, wobei deren Tiefe und die Höhe des Halteelements (34) des Einwurföffnungsdeckels (30) aneinander angepasst sind.

3. Behälter nach Anspruch 2, **dadurch gekennzeichnet, dass** die Vertiefung (10) und daran angepasst auch die Einwurföffnung (20) eine elliptische Form aufweisen.

4. Behälter nach Anspruch 3, **dadurch gekennzeichnet, dass** die elliptische Vertiefung (10) so angeordnet ist, dass sie im Bereich eines ihrer Nebenscheitel an die Randkante des Behälterdeckelteils (2) angrenzt.

5. Behälter nach Anspruch 4, **dadurch gekennzeichnet, dass** bei einem quadratischen Behälterdeckelteil (2) dieser Nebenscheitel nahe der Randkante der als Stirnseite vorgesehenen Seite des Behälterdeckelteils (2) endet.

6. Behälter nach Anspruch 5, **dadurch gekennzeichnet, dass** im Bereich des gegenüberliegenden Nebenscheitels die Vertiefung (10) durch eine eckige Erweiterung (11) vergrößert ist, auf deren Boden (13) zwei sich annähernd bis zur Oberseite des Behälterdeckelteils (2) erstreckende Haltenasen (39) für die klappbare Anordnung des Einwurföffnungsdeckels (30) ausgebildet sind.

7. Behälter nach einem der Ansprüche 2 bis 6, **dadurch gekennzeichnet, dass** die Seitenwandung (12) der Vertiefung (10) querschnittsverengend schräg einwärts gerichtet nach unten verläuft.

8. Behälter nach einem der vorhergehenden Ansprüche, **dadurch gekennzeichnet, dass** als Einrastelemente (33) des Verschlusskragens (32) auf seiner Außenseite, vom unteren Endbereich ausgehend, eine Anzahl jeweils voneinander beabstandeter Einrastzungen schräg nach oben gerichtet abstehend ausgebildet sind.

9. Behälter nach Anspruch 8, **dadurch gekennzeichnet, dass** das Halteelement (34) in Anpassung an die Ausgestaltung der Verriegelungszungen (33) des Verschlusskragens (32) zwischen seinem oberen und unteren Ende einen querschnittsverengend schräg einwärts gerichtet verlaufenden Wandungsabschnitt (35) aufweist.

10. Behälter nach Anspruch 9, **dadurch gekennzeichnet, dass** auf der Innenseite des Halteelements (34) am oberen Rand eine zum Zusammenwirken mit den Verriegelungszungen (33) bestimmte Rastlippe (36) nach innen vorspringt.

11. Behälter nach einem der Ansprüche 8 bis 10, **dadurch gekennzeichnet, dass** in Anpassung an die schräg nach oben gerichtet außen vom Verschlusskragen (32) abstehenden Einrastzungen (33) der äußere Schenkel (23) des Sitzes (22) im elliptischen Bereich der Vertiefung (10) in entsprechend schräger Verlängerung von deren schräg verlaufender Seitenwandung (12) und im Bereich der eckigen Erweiterung (11) der Vertiefung (10) dementsprechend ausgebildet vom Rand deren Bodens (13) absteht und oben an seiner Randkante als Sitz-Verriegelungselement (24) für die Einrastzungen (33) eine geringfügig vorspringende Verriegelungslippe aufweist, während der demgegenüber in Anpassung an die die Einwurföffnung (20) endgültig verschließende Anordnung des Deckelelements (31) kürzere innere Schenkel (25) des Sitzes (22) senkrecht hoch steht und an seinem oberen Ende eine nach innen vorspringende Verstärkungsrippe (26) aufweist.

12. Behälter nach einem der vorhergehenden Ansprüche, **dadurch gekennzeichnet, dass** für den Sitz (11) eine Dichtung vorgesehen ist.

13. Behälter nach einem der Ansprüche 5 bis 12, **dadurch gekennzeichnet, dass** entsprechend dem Abstand der Haltenasen (39) voneinander zwei Halterungen (38) seitwärts vom Rand des Halteelements (34) abstehen, jeweils gebildet von zwei geringfügig voneinander beabstandeten, im sich verjüngenden freien Endbereich jeweils durch einen Achssteg mit rundem Querschnitt miteinander verbundenen Haltezungen, welche Achsstege jeweils in eine entsprechende Ausnehmung in den Haltenasen (39) eingerastet sind.

14. Behälter nach einem der vorhergehenden Ansprüche, **dadurch gekennzeichnet, dass** der Einwurföffnungsdeckel (30) in seiner zugeklappten Stellung durch eine Arretierung (41, 42, 43) in der Vertiefung (10) gehalten ist.

15. Behälter nach Anspruch 14, **dadurch gekennzeichnet, dass** zur Arretierung des Einwurföffnungsdeckels (30) in dem der Mitte zwischen den beiden Haltenasen (39) gegenüberliegenden Randbereich des Behälterdeckelteils (2) eine die Vertiefung (10) nach außen erweiternde Arretierungsausnehmung (40) mit einer senkrecht hoch stehenden, nach außen auslenkbaren Arretierungsraste (41) mit einer Arretierungskerbe (42) auf der Innenseite ausgebildet ist, die mit einer vom Außenrand des Halteelements (34) nach außen abstehenden Arretierungslasche (43) zusammenwirkt.

16. Behälter nach Anspruch 14 oder 15, **dadurch gekennzeichnet, dass** der Einwurföffnungsdeckel (30) bzw. das "leere" Halteelement (34) durch Federkraft ihre aufgeklappte Stellung einnehmen.

## Claims

1. Collection and disposal receptacle, in particular for medical waste, having a lower receptacle portion (1) and a receptacle lid portion (2) with an insertion opening (20) and a plate-like insertion-opening lid (30) which covers the edge (21) of the insertion opening (20) and can be pivoted open and shut, co-operating closure and latch elements (22, 33) for final closure of the insertion opening (20) in a manner inaccessible from the outside being provided in each case in the edge region of insertion opening (20) and insertion-opening lid (30), **characterised** at least
- in that the insertion-opening lid (30) is composed of two parts:
a) a lid element (31) intended for final closure of the insertion opening (20) and having a plate-like shape, with a shape adapted to the contour of the edge (21) and the inside width of the insertion opening (20), having a closure collar (32) which projects downwardly at the edge and on the outer side of which, increasing its circumference, projecting laterally in spring fashion and capable of being pressed against this outer side so as to reduce the circumference, are formed the latch elements (33), and
b) a holding element (34) which holds the lid element (31) until final closure of the insertion opening (20) and is arranged on the receptacle lid portion (2) so as to be pivotable open and shut with the lid element (31), and which has a shape enabling it to rest on the receptacle lid portion (2) in the edge region of the insertion opening (20) and surrounding the lid element (31) at the edge like a frame, the latch elements (33) of the lid element in this case abutting against the inner side of the holding element (34) under tension in such a way that, when the holding element (34) rests on the receptacle lid portion (2), the lid element (31) can be pushed downwardly out of the holding element (34) by applying force, and
- in that the edge of the insertion opening (20) as the closure element has a peripheral, approximately U-shaped seat (22) for the closure collar (32) of the lid element (31) which is pushed out downwardly, the seat projecting downwardly at least in one section and protruding into the insertion opening (20) and having a seat locking element (24) co-operating with the latch elements (33) of the lid element (31) for final closure of the insertion opening (20).

2. Receptacle according to claim 1, **characterised in that** the insertion opening (20) is arranged in a flat depression (20) formed like a trough in the upper side of the receptacle lid portion (2), the depth thereof and the height of the holding element (34) of the insertion-opening lid (30) being adapted to each other.

3. Receptacle according to claim 2, **characterised in that** the depression (10) and, adapted thereto, the insertion opening (20) as well have an elliptical shape.

4. Receptacle according to claim 3, **characterised in that** the elliptical depression (10) is arranged so as to adjoin the edge of the receptacle lid portion (2) in the region of one of its minor vertices.

5. Receptacle according to claim 4, **characterised in that**, in the case of a square receptacle lid portion (2), this minor vertex ends close to the edge of the side of the receptacle lid portion (2) which is provided as the front side.

6. Receptacle according to claim 5, **characterised in that**, in the region of the opposite minor vertex, the depression (10) is enlarged by an angular extension (11) on the bottom (13) of which are formed two holding lugs (39) extending approximately as far as the upper side of the receptacle lid portion (2) for the pivotable arrangement of the insertion-opening lid (30).

7. Receptacle according to any of claims 2 to 6, **characterised in that** the side wall (12) of the depression (10) extends downwardly, directed obliquely inwards so as to narrow the cross-section.

8. Receptacle according to any of the preceding claims, **characterised in that** a number of latch tongues spaced apart from each other are formed, projecting obliquely upwards, as latch elements (33) of the closure collar (32) on its outer side, extending from the lower end region.

9. Receptacle according to claim 8, **characterised in that** the holding element (34) has a wall section (35) directed obliquely inwards so as to narrow the cross-section in adaptation to the shape of the locking tongues (33) of the closure collar (32) between its upper and lower ends.

10. Receptacle according to claim 9, **characterised in that**, on the inner side of the holding element (34) at the upper edge, a latch lip (36) designed for cooperation with the locking tongues (33) projects inwards.

11. Receptacle according to any of claims 8 to 10, **characterised in that**, in adaptation to the latch tongues (33) which project obliquely upwards from the closure collar (32) on the outside, the outer arm (23) of the seat (22) in the elliptical region of the depression (10) projects in a correspondingly oblique extension from its obliquely extending side wall (12) and in the region of the angled extension (11) of the depression (10) projects, shaped correspondingly, from the edge of the bottom (13) thereof, and at the top at its edge has a slightly protruding locking lip as a seat locking element (24) for the latch tongues (33), while the inner arm (25) of the seat (22), which is shorter by comparison in adaptation to the arrangement of the lid element (31) which finally closes the insertion opening (20), is perpendicularly upright and at its upper end has an inwardly protruding reinforcing rib (26).

12. Receptacle according to any of the preceding claims, **characterised in that** a seal is provided for the seat (11).

13. Receptacle according to any of claims 5 to 12, **characterised in that**, matching the distance between the holding lugs (39), two supports (38) project laterally from the edge of the holding element (34), in each case formed by two slightly spaced-apart holding tongues connected to each other in the tapering free end region in each case by an axial web of round cross-section, which axial webs are in each case latched in a corresponding recess in the holding lugs (39).

14. Receptacle according to any of the preceding claims, **characterised in that** the insertion-opening lid (30) in its shut position is held in the depression (10) by a catch (41, 42, 43).

15. Receptacle according to claim 14, **characterised in that**, for catching of the insertion-opening lid (30) in the edge region of the receptacle lid portion (2) opposite the centre between the two holding lugs (39), a catch recess (40) which increases the width of the depression (10) outwardly with a perpendicularly upright, outwardly deflectable catch means (41) having a catch notch (42) is formed on the inner side, which co-operates with a catch strip (43) projecting outwards from the outer edge of the holding element (34).

16. Receptacle according to claim 14 or 15, **characterised in that** the insertion-opening lid (30) or the "idle" holding element (34) occupy their open position by spring force.

## Revendications

1. Conteneur de collecte et d'élimination, en particulier pour déchets médicaux, avec une partie inférieure de conteneur (1) et une partie de couvercle de conteneur (2), avec une ouverture d'introduction (20) et un couvercle d'ouverture d'introduction (30) du genre d'une plaque couvrant le bord (21) de l'ouverture d'introduction (20) disposé susceptible d'être ouvert et fermé par rabattement, des éléments de fermeture et d'encliquetage (22, 33), coopérant pour assurer la fermeture définitive de l'ouverture d'introduction (20) inaccessible depuis l'extérieur, étant chaque fois prévus dans la zone de bordure de l'ouverture d'introduction (20) et du couvercle d'ouverture d'introduction (30),
**caractérisé** au moins en ce que
- le couvercle d'ouverture d'introduction (30) est formé de deux parties :
a) un élément de couvercle (31) déterminé pour la fermeture définitive de l'ouverture d'introduction (20), présentant une configuration du genre d'une plaque, avec une configuration adaptée au contour du bord (21) et au diamètre intérieur de l'ouverture d'introduction (20), avec une collerette de fermeture (32) située côté bordure et faisant saillie vers le bas, sur la face extérieure de laquelle, en agrandissant sa circonférence, les éléments d'encliquetage (33), faisant saillie élastiquement latéralement et susceptibles d'être pressés en diminuant la circonférence sur cette face extérieure sont réalisés, et
b) un élément de maintien (34) maintenant l'élément de couvercle (31) jusqu'à la fermeture définitive de l'ouverture d'introduction (20), disposé avec lui sur la partie de couvercle de conteneur (2) en étant susceptible d'être ouvert et fermé par rabattement, élément de maintien qui, a une configuration en forme de cadre entourant, côté bordure, l'élément de couvercle (31), permettant sa pose sur la partie de couvercle de conteneur (2) dans la zone de bordure de l'ouverture d'introduction (20), les éléments d'encliquetage (33) de l'élément de couvercle étant en appui ici sous contrainte sur la face intérieure de l'élément de maintien (34), de manière que, lorsque l'élément de maintien (34) repose sur la partie de couvercle de conteneur (2), l'élément de couvercle (31) puisse être sorti vers le bas hors de celui-ci, en exerçant une pression par déploiement de force, et
- en ce que le bord de l'ouverture d'introduction (20) présente, en tant qu'élément de fermeture, un siège (22) à peu près en forme de U, faisant le pourtour au moins par tronçon en saillie vers le bas et projetant dans l'ouverture d'introduction (20), pour la collerette de fermeture (32) de l'élément de couvercle (31) ressorti vers le bas par pressage, le siège présentant un élément de verrouillage de siège (24) coopérant avec les éléments d'encliquetage (33) de l'élément de couvercle (31) pour produire la fermeture définitive de l'ouverture d'introduction (20).

2. Conteneur selon la revendication 1, **caractérisé en ce que** l'ouverture d'introduction (20) est disposée dans une cavité (10) plate en forme d'auge, réalisée dans la face supérieure de la partie de couvercle de conteneur (2), la profondeur de la cavité et la hauteur de l'élément de maintien (34) du couvercle d'ouverture d'introduction (30) étant adaptées l'une à l'autre.

3. Conteneur selon la revendication 2, **caractérisé en ce que** la cavité (10) et, lui étant adaptée, également l'ouverture d'introduction (20), présentent une forme elliptique.

4. Conteneur selon la revendication 3, **caractérisé en ce que** la cavité (10) elliptique est disposée de manière qu'elle avoisine l'arête de bordure de la partie de couvercle de conteneur (2) dans la zone d'un de ses sommets annexes.

5. Conteneur selon la revendication 4, **caractérisé en ce que**, dans une partie de couvercle de conteneur (2) carrée, ce sommet annexe s'achève à proximité de l'arête de bordure du côté, prévu comme face frontale, de la partie de couvercle de conteneur (2).

6. Conteneur selon la revendication 5, **caractérisé en ce que**, dans la zone du sommet annexe opposé, la cavité (10) est agrandie par un élargissement (11) anguleux, au fond (13) duquel sont réalisés deux ergots de maintien (39) s'étendant à peu près jusqu'à la face supérieure de la partie de couvercle de conteneur (2), pour l'agencement rabattable du couvercle d'ouverture d'introduction (30).

7. Conteneur selon l'une des revendications 2 à 6, **caractérisé en ce que** la paroi latérale (12) de la cavité (10) s'étend, en rétrécissant la section transversale, obliquement vers l'intérieur vers le bas.

8. Conteneur selon l'une des revendications 2 à 6, **caractérisé en ce qu'**une pluralité de languettes d'encliquetage, espacées chaque fois les unes des autres, sont réalisées en étant orientées obliquement vers le haut et en saillie, en tant qu'éléments d'encliquetage (33) de la collerette de fermeture (32) sur sa face extérieure, en partant de la zone d'extrémité inférieure.

9. Conteneur selon la revendication 8, **caractérisé en ce que** l'élément de maintien (34), en adaptation à la configuration des languettes de verrouillage (33) de la collerette de fermeture (32), entre son extrémité supérieure et son extrémité inférieure, présente une section de paroi (35) s'étendant obliquement vers l'intérieur et rétrécissant la section transversale.

10. Conteneur selon la revendication 9, **caractérisé en ce que** sur la face intérieure de l'élément de maintien (34), sur le bord supérieur, une languette d'encliquetage (36), conçue pour coopérer avec les languettes de verrouillage (33) fait saillie vers l'intérieur.

11. Conteneur selon l'une des revendications 8 à 10, **caractérisé en ce que**, en adaptation avec les languettes d'encliquetage (33), faisant saillie extérieurement de la collerette de fermeture (32) et orientées obliquement vers le haut, la branche extérieure (23) du siège (22) fait saillie, dans la zone elliptique de la cavité (10), en un prolongement oblique correspondant de sa paroi latérale (12) s'étendant obliquement et fait saillie du bord de son fond (13), dans la zone de l'élargissement (11) anguleux de la cavité (10), en étant configurée de manière appropriée et en partie supérieure sur son arête de bordure, en tant qu'élément de verrouillage de siège (24) pour les languettes d'encliquetage (33), présente une lèvre de verrouillage dépassant légèrement, tandis que la branche intérieure (25) du siège (22) plus courte, en adaptation à l'agencement, fermant définitivement l'ouverture d'introduction (20), de l'élément de couvercle (31), monte verticalement et présente, à son extrémité supérieure, une nervure de renforcement (26) dépassant vers l'intérieur.

12. Conteneur selon l'une des revendications précédentes, **caractérisé en ce qu'**un joint d'étanchéité est prévu pour le siège (11).

13. Conteneur selon l'une des revendications 5 à 12, **caractérisé en ce que**, de manière correspondante à l'espacement mutuel des ergots de maintien (39), deux fixations (38) font saillie latéralement du bord de l'élément de maintien (34), chacune formée par deux languettes de maintien légèrement espacées l'une de l'autre, reliées ensemble, dans la zone d'extrémité libre s'effilant, chaque fois par une traverse d'axe à section transversale ronde, les traverses d'axe étant encliquetées chacune dans un évidement correspondant ménagé dans les ergots de maintien (39).

14. Conteneur selon l'une des revendications précédentes, **caractérisé en ce que** le couvercle d'ouverture d'introduction (30), à sa position fermée par rabattement, est maintenu dans la cavité (10) par un élément d'arrêt (41, 42, 43).

15. Conteneur selon la revendication 14, **caractérisé en ce que**, pour bloquer le couvercle d'ouverture d'introduction (30) dans la zone de bordure opposée au centre entre les deux ergots de maintien (39) de la partie de couvercle de conteneur (2), un évidement d'arrêt (40) élargissant vers l'extérieur de la cavité (10), avec un cran d'arrêt (41) susceptible de s'écarter vers l'extérieur et faisant saillie vers le haut, verticalement, est réalisé avec une entaille d'arrêt (42) en face intérieure, qui coopère avec une patte de blocage (43) faisant saillie vers l'extérieur, depuis le bord extérieur de l'élément de maintien (34).

16. Conteneur selon la revendication 14 ou 15, **caractérisé en ce que** le couvercle d'ouverture d'introduction (30) respectivement l'élément de maintien "vide" (34) prennent leur position ouverte par rabattement grâce à la force d'un ressort.
